# EUROPEAN PATENT SPECIFICATION

(11) **EP 1 654 992 B1**
(45) Date of publication and mention of the grant of the patent: **02.01.2008**
(21) Application number: 06001493.3
(22) Date of filing: 07.02.2002
(51) Int. Cl.: A61B 17/122, A61B 17/128

(54) **Deployable surgical clamp with delivery/retrieval device and actuator**
Ausschwenkbare chirurgische Klammer mit einer Installations-/Entnahmevorrichtung und Bedienelement
Clamp chirurgical déployable avec dispositif de pose/récupération et actionneur

(30) Priority: 07.02.2001 US 266930 P; 01.10.2001 US 968101
(43) Date of publication of application: 10.05.2006
(62) Divisional of application: 02002777.7
(73) Proprietor: Karl Storz Endoscopy-America, Inc., Culver City, CA 90230-7600 (US)
(72) Inventor: Solingen, Simon, 90077 Los Angeles CA 90077 (US)
(74) Representative: Heuckeroth, Volker

(56) References cited:
- US-A- 4 651 737
- US-A- 5 855 590
- US-A- 5 921 996
- US-A- 6 146 394
- US-A1- 2001 049 540

## Description

The present invention relates to surgical clamp system for endoscopic or open surgery.

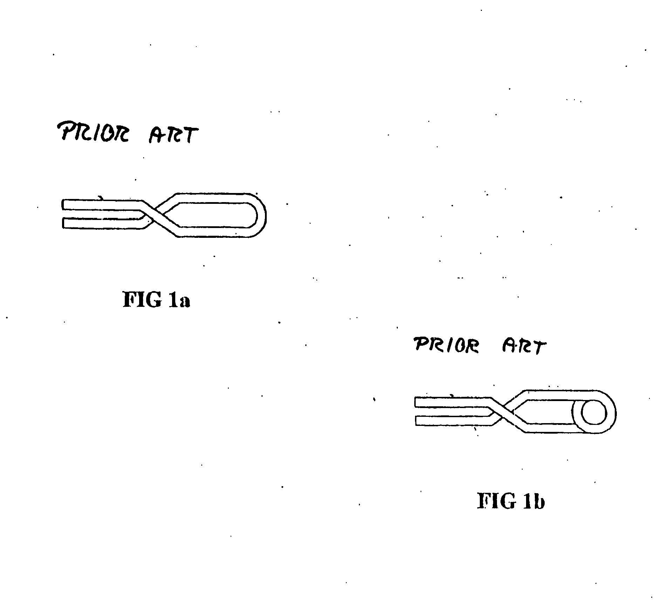

Surgical clamps that can be detached from their dedicated actuators have been available for some time. These clamps are typically spring loaded, i.e. they depend upon the force of an integral spring to remain closed, and are opened by "squeezing" the proximal end of the jaw assemblies (Refer to Prior Art, FIG 1a and FIG 1 b). Clamp delivery devices consist primarily of dedicated "pliers type" actuators that squeeze the clamp open, place the clamp at the desired location, and then release the clamp which then closes under integral spring pressure.

During certain surgical procedures, it may be necessary to temporarily and securely occlude body conduits (such as blood vessels and the like) of relatively large size, which due to disease, may have uneven wall thickness, or may be partially obstructed or stenotic. In open surgery, when access to the site is not limited, standard vascular type clamps (utilizing ring handles) are typically applied. Clamping force is increased until the body conduits are effectively and securely occluded, and then locked by means of ratchets. In endoscopic surgery, clamps are typically introduced to the surgery site through access ports (small openings made by a surgeon, and/or through natural existing body openings). However, it is desirable to reduce the number of openings made by the surgeon, as well as to reduce instrument clutter at the surgical site itself. Standard vascular type clamps do not lend themselves to reducing the number of surgical access ports and/or reducing instrument clutter at a surgical site. Spring loaded clamps have the limitation of not delivering sufficient clamping force to overcome the non-uniform thickness of body conduit walls to provide effective occlusion. Additionally, spring loaded clamps have a tendency of dislodging (siipping off) when disturbed, due to insufficient clamping force, thus risking patient safety. The tendency of dislodging (slipping off) applies, as well, when clamps are used to temporarily retract tissue.

The jaws of spring-loaded clamps are normally fully closed (exacting full clamping force as provided by spring tension) when detached from their delivery device, thus the clamping force cannot be varied by the surgeon. In order to apply differing clamping forces necessary for diverse medical procedures, many clamps with identical jaws and different spring tension must be available and used. One variable related to clamp effectiveness is tissue thickness (the thicker the tissue, the greater the resulting clamping force). Clamping force is limited by the spring tension, which is derived from the spring's physical size. If the spring's physical size is large to provide greater clamping force, other aspects of surgery must be considered. Access ports typically have small diameters (the overall philosophy of endoscopic surgery is to make as few access port openings as possible, with the smallest diameter as possible), thus the larger size springs required for greater clamping force are difficult to pass through the access port(s) to the surgical site. Additionally, the ability of the delivery method to squeeze the clamp open is limited; the greater spring tension required, the more difficult it becomes to remotely open the clamp.
Document US-A-5 749 881 represents the closet prior art and discloses a surgical clamp combination similar in construction to the device of claim 1, including a clamp movable between a free state and operative state, the clamp including a pair of jaws which can be operated to occlude a body conduit. A clamp applier is adapted to releasibly engage the clamp in the free state and to operate the clamp in the operative state to close the jaws. The applier has a housing disposed at a proximal end and a tube extending distally and configured to receive the clamp in the operable state. A shaft is disposed within the tube and movable between an extended position to engage the clamp in the free state and a retracted position to releasibly hold the clamp in the operable state. With the clamp in the operable state, the shaft is rotatable by operation of a handle and aladdin screw, to alternatively rotate in opposite directions to open and close the jaws of the clamp.
From document US-A-5 855 590 a clamp for clamping a body structure in a patient is known. In one embodiment of this known clamp system, the clamp includes first and second jaws having opposed jaw surfaces. The clamp is releasibly connected to a clamp positioner. Further, the clamp includes an actuator housing slideably mounted to an extension of one of the jaw parts. The slideable actuator housing moves the jaw between open and closed positions through a pin and slot configuration. Movement of the actuator housing in axial direction causes the jaw to pivot upwardly from the close position and vice versa. The clamp positioner of this known clamp includes an elongate hollow body sized to fit within a trocar sleeve with the clamp mounted to the distal end of the clamp positioner. The elongate hollow body of the clamp positioner houses a pair of longitudinally slideable manipulator rods coupled to handgrips. Each of the manipulator rods has a lock which engages L-shaped slots formed in the actuator housing and in the extension of one of the jaw parts. By virtue of that configuration, the clamp positioner functions as an actuator which is directly attached with the clamp via the actuator housing of the clamp.
Document US-A-5 921 996 discloses a surgical clamp applier and a detachable clamp for temporarily occluding a vessel. The clamp applier has a dial, a button, and a pulley actuator all accessible on the handle of the applier. The dial is used to lock the clamp onto the applier and to remove the clamp from the applier. The button is used to allow the pulley actuator to be used to manipulate the lower jaw of the clamp with respect to the upper jaw of the clamp, thus varying the amount of pressure exerted by the clamp on the vessel. The pulley actuator actuates the pulley system which engages the clamp, thereby manipulating the lower jaw of the clamp with respect to the upper jaw in a scissor-like manner.
Document US-A-6 146 394 discloses a vascular clamp assembly which includes at least one bendable elongated shaft. A pair of clamping members are located at the distal part of the shaft. The clamp includes means for moving the clamping members between an open position and a clamping position.
What is desired, is a surgical clamp which delivers sufficient clamping force to overcome the non-uniform thickness of body conduit walls to provide effective occlusion, which does not have a tendency of dislodging (slipping off) when disturbed, which allows the clamping force to be varied by the surgeon, and which provides a clamping force which is not limited by spring tension.
Accordingly, it is an object of the present invention to provide a surgical clamp which delivers sufficient clamping force to overcome the non-uniform thickness of body conduit walls to provide effective occlusion.

Another object of the present invention is to provide a surgical clamp having the above characteristics and which does not have a tendency of dislodging (slipping off) when disturbed.

A further object of the present invention is to provide a surgical clamp having the above characteristics and which allows the clamping force to be varied by the surgeon.

Still another object of the present invention is to provide a surgical damp having the above characteristics and which provides a clamping force which is not limited by spring tension.

These and other objects of the present invention are achieved by provision of a surgical clamp system including a clamp, a delivery/retrieval device, and an actuator according to claim 1. The clamp includes a pair of jaws actuatable relative to each other from a fully open to a fully closed position. The delivery/retrieval device is detachably connected to the clamp, and is operable by a surgeon to deploy the clamp, retrieve the clamp, or both. The actuator cooperates with the delivery/retrieval device and engages the clamp, and is operable by the surgeon to open and close the jaws of the clamp. The delivery/retrieval device and the actuator are operable to securely lock the jaws of the clamp in any position between the fully open and the fully closed positions, both during and after being detached from the delivery/retrieval device.

The pair of jaws of the clamp may be configured such that both are movable, or such that one is movable and the other is stationary. In one embodiment, the pair of jaws of the clamp may be substantially parallel to each other in any position between the fully open and the fully closed positions.
The clamp includes, for actuating and/or locking the jaws, a worm gear/drive gear arrangement.
The delivery/retrieval device includes an elongated hollow shaft, a distal end of which is detachably to the clamp, and a proximal end of which is adapted to receive the actuator. The delivery/retrieval device is detachably connectable to the clamp by a bayonet connection.

The invention and its particular features and advantages will become more apparent from the following detailed description considered with reference to the accompanying drawings.

**Figs. 1a** and **1b** are side views of prior art surgical clamps that can be detached from their dedicated actuators;

**Fig**. **2a** is a side view, partially in section, of a deployable clamp, shown with its jaw closed;

**Fig. 2b** is a side view, partially in section, of the deployable clamp of FIG. 2a, shown with its jaw opened;

Fig. 2c is an end view of the deployable clamp of FIG. 2a;

**Fig. 3a** is a side view of another embodiment of a deployable clamp which may be in accordance with the present invention, shown with its jaw closed;

**Fig. 3b** is a side view of the deployable clamp of FIG. 3a, shown with its jaw opened;

**Fig. 3c** is an end view of the deployable clamp of FIG. 3a;

**Fig. 4a** is a side view of one embodiment of a delivery/retrieval device in accordance with the present invention;

**Fig. 4b** is an end view of the delivery/retrieval device of FIG. 4a;

**Fig. 5a** is a side view of one embodiment of an actuator in accordance with the present invention;

**Fig. 5b** is an end view of the actuator of FIG. 5a;

**Fig. 6** is a side view illustrating the actuator of FiG. 5a inserted into the proximal end of the delivery/retrieval device of FIG. 4a; and

**Fig. 7** is a side view illustrating the clamp of FIG. 2a attached to the delivery/retrieval device of FIG. 4a, with the actuator of FIG. 5a latched in place.

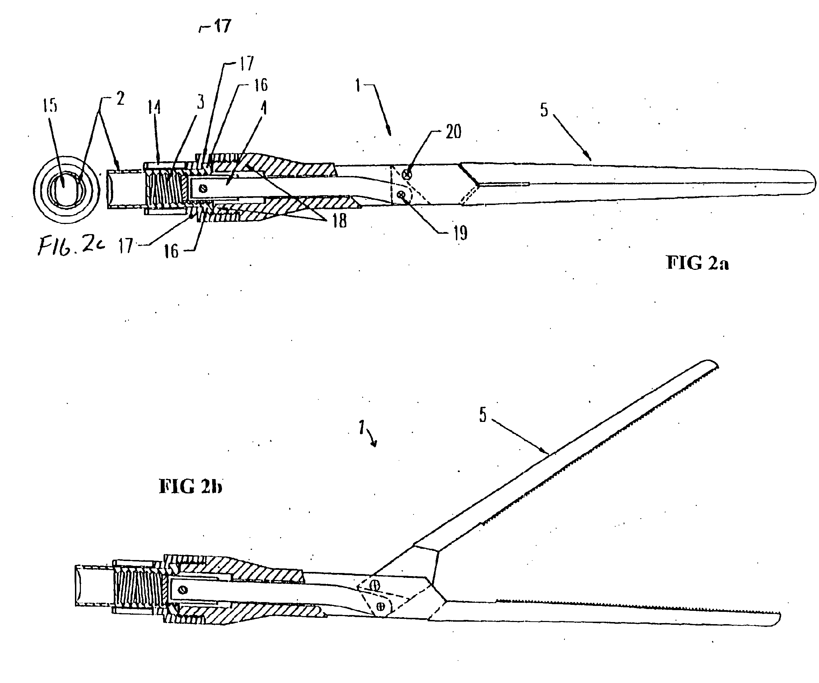

Referring first to FIGS. 2a and 2c, one embodiment of a deployable clamp **1** is illustrated, shown with Jaw **5** closed. FIG 2b depicts the clamp **1** with jaw **5** open. Externally accessible nut **2** is threaded over screw **3** and prevented from axial displacement by flange **16** kept rotatably between bushing **17** and clamp body **18.** Screw **3** is permanently coupled to push-pull rod **4,** which is engaged to jaw **5** by means of articulation **19.** Rotating nut **2** causes axial displacement of screw **3,** which puils or pushes on the jaw **5** by means of push-pull rod **4** acting over articulation **19,** causing jaw **5** to articulate about pivot **20.** It should be understood that while a screw/nut drive is described in the detailed example presented herein, the screw and nut can be interchanged (i.e. the nut can be attached to the push-pull rod), and that other configurations for actuating the jaws are also possible. According to an embodiment of the invention, nut **2** is replaced by a worm gear and screw **3** is replaced with a rack gear which cooperates with the worm gear such that jaws are actuated by action of the gear system. In another arrangement, nut **2** can be replaced by a worm gear and the proximal end(s) of the movable jaw(s) be configured as a gear(s) sector(s) which cooperate(s) with the worm gear such that jaws are actuated by action of the gear system.

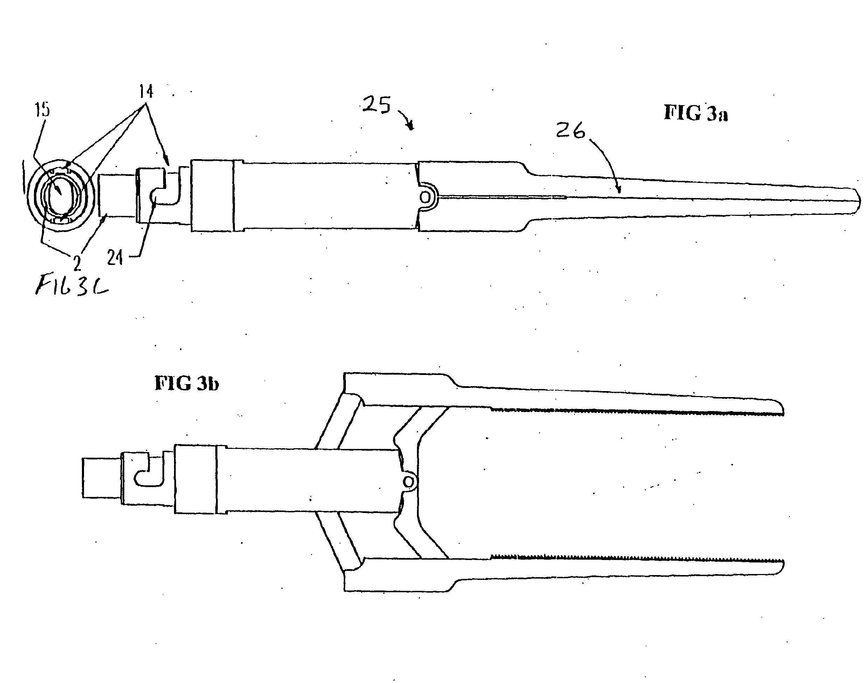

FIGS. 3a and 3c depict another embodiment of the deployable clamp **25,** where two jaws are articulated and can open and close apposing (parallel to) each other **26.** FIG. 3b depicts clamp **25** with both jaws open. The mechanism to open and close both jaws is identical to that described in FIG 2a and 2b. Various jaw types (long, short, curved, etc.) can be utilized with the described clamping mechanism as required depending upon the surgical procedure being performed.

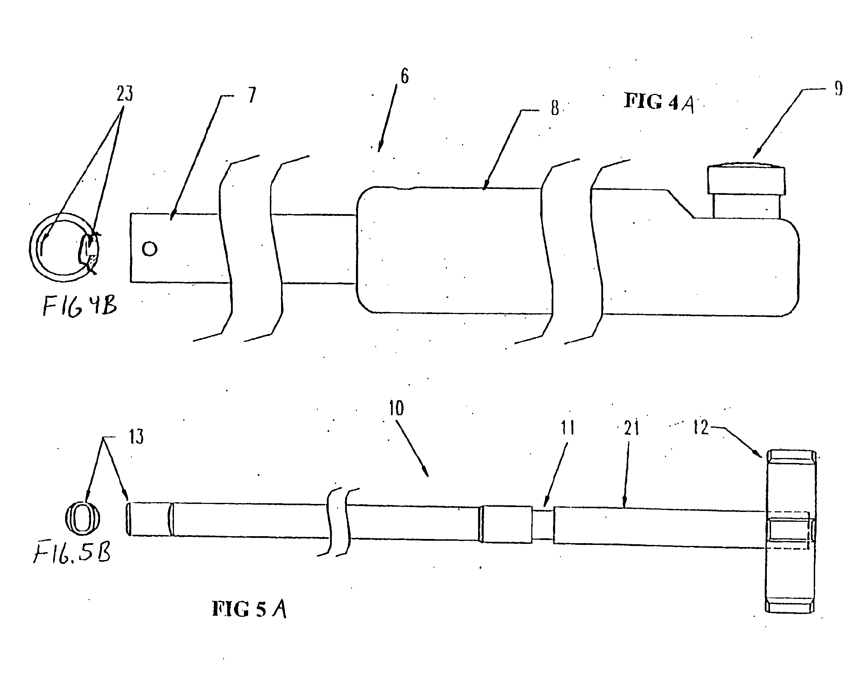

Referring now to FIGS. 4a and 4b, one embodiment of the delivery/retrieval device **6** is shown comprising a hollow shaft **7,** handle **8,** and a spring loaded latching mechanism (not shown) that can be released by push-button **9.**

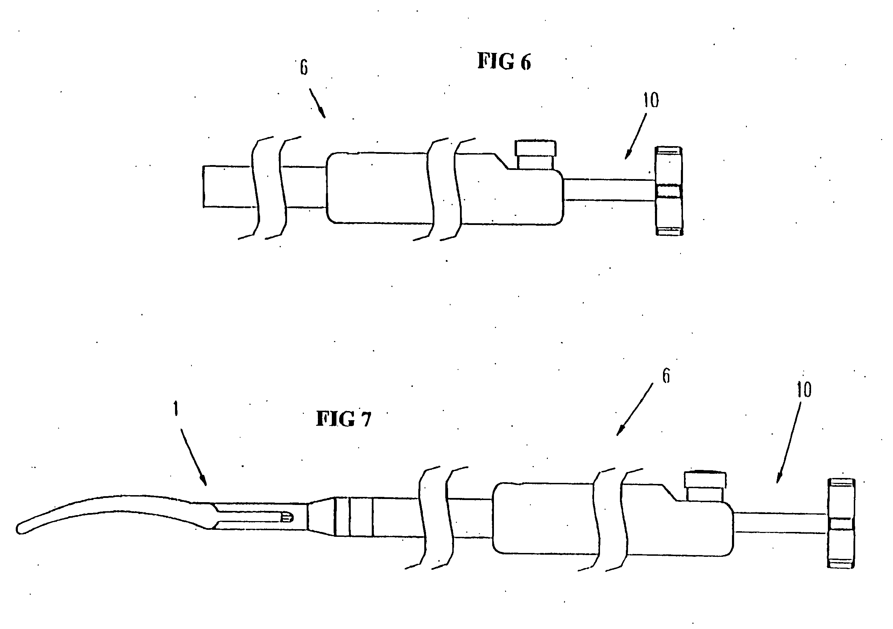

FIGS. 5a and 5b depict one embodiment of an actuator **10** comprising a shaft **21** with proximal knob **12,** and groove **11** which, when engaged by spring loaded latching mechanism (not shown), prevents shaft **21** from displacing axially. Distal end **13** of shaft **21** engages slot **15** (FIG 2c) in nut **2** (FIG 2c) of clamp **1** or clamp **25** (FiG. 3c).

FIG. 6 depicts actuator **10** inserted into the proximal end of delivery/retrieval device **6,** while FIG. 7 depicts clamp **1** attached to delivery/retrieval device **6,** with actuator **10** latched in place.

The following describes a typical use of either clamp **1** or clamp **25** similar to that according to the present invention, delivery/retrieval device **6**, and actuator **10**.

The proximal end of clamp **1, 25** is inserted into distal end of hollow shaft **7** and rotated to engage and lock bayonet pins **23** inside distal end of hollow shaft **7** of deliverylretrieval device **6** to bayonet **14** (FIG. 2a and FIG. 3a). While holding handle **8,** actuator **10** is introduced into the proximal end of handle **8,** and pushed through and simultaneously rotated in hollow shaft **7** until distal end **13** penetrates and engages slot **15** of nut **2,** at which point spring loaded latch mechanism (not shown) engages grove **11** and locks actuator **10** in place. Bayonet **14** is provided with axial grooves **24** (FIG. 3a) which are engaged to pins **23** (FIGS. 4a and 4b) when actuator **10** is locked in place thus pushing against the bottom of slot **15.** Clamp **1** is prevented from rotating with respect to hollow shaft **7,** which keeps the clamp securely locked place. Clamp **1** is prevented from being removed from hollow shaft **7** until latch (not shown) is disengaged from groove **11** and actuator **10** is withdrawn.

The assembled system is introduced into the surgical site, jaw **5** is opened by rotating knob **12,** while holding handle **8** stationery. The open clamp is placed over the desired tissue, and jaw **5** is closed by rotating knob **12** in the opposite direction, while holding handle **8** stationery. Tactile feedback, representative of the delivered clamping force, is provided to the surgeon via the resistance to rotation felt on knob **12.** Once the desired clamping force is achieved, push button **9** is pressed releasing actuator shaft **21.** Actuator **10** can then be removed from delivery/retrieval device **6**.

Delivery/retrieval device **6** is removed from deployed clamp **1** by use of a grasping means (i.e. a pair endoscopic hemostats or the like) to hold clamp **1** stationery, while handle **8** is rotated to disengage bayonet pins **23** from axial grooves **14.** Delivery/retrieval device **6** can then be removed from the surgery site, as well as the employed grasping means used during clamp and delivery/retrieval device separation.

When deployed clamp **1** is no longer required, delivery/retrieval device **6** is reintroduced into the surgical site, and by use of a grasping means, clamp **1** is held stationery. Distal end of hollow shaft **7** is slipped over the proximal end of clamp **1** and handle **8** is simultaneously pushed and rotated to engage bayonet pins **23** with bayonet **14.** While holding handle **8,** actuator **10** in introduced into the proximal end of handle **8,** and pushed through and simultaneously rotated in hollow shaft **7** until distal end **13** penetrates and engages slot **15** of nut **2,** at which time latch (not shown) engages grove **11** and locks actuator **10** in place. Knob **12** is then rotated, while handle **8** is held stationery, to open jaw **5** of clamp **1.** Once jaw **5** is open, clamp **1** is pulled away from the tissue. Knob **12** is rotated, while handle **8** is held stationery, until jaw **5** is fully closed, then the entire assembly can be withdrawn from the surgical site.

The present invention, therefore, provides a surgical clamp which delivers sufficient clamping force to overcome the non-uniform thickness of body conduit walls to provide effective occlusion, which does not have a tendency of dislodging (slipping off) when disturbed, which allows the clamping force to be varied by the surgeon, and which provides a clamping force which is not limited by spring tension.

## Claims

1. A surgical clamp system comprising:
a clamp (1; 25) having a pair of jaws (5; 26) actuatable relative to each other from a fully open to a fully closed position;
a delivery/retrieval device (6) detachably connected to said clamp (1; 25) by a bayonet connection (14; 23), wherein said delivery/retrieval device (6) has a hollow shaft (7) with a longitudinal opening there-through, a distal portion of the opening configured to engage with said clamp (1; 25), said delivery/retrieval device (6) being operable by a surgeon to perform a function selected from the group comprising deploying said clamp (1; 25), retrieving said clamp (1; 25), or both;
an actuator (10) cooperating with said delivery/retrieval device (6), said actuator (10) being at least partially insertable through the longitudinal opening of said delivery/retrieval device (6) for engaging said clamp (1; 25) and being operable by the surgeon to open and close the jaws (5; 26) of said clamp (1; 25); and
wherein said delivery/retrieval device (6) and said actuator (10) are operable to securely lock the jaws (5; 26) of said clamp (1; 25) in any position between the fully open and the fully closed positions, both during and after being detached from said delivery/retrieval device, wherein either said clamp comprises:
a push-pull rod connected at one end to at least one of the pair of jaws (5; 26) of said clamp;
a rack gear connected to an end of said push-pull rod opposite to the end connected to at least one of the pair of jaws of said clamp;
a worm gear engaging said rack gear, said worm gear being rotatable with respect to said rack gear in order to cause axial displacement of said rack gear and actuation of the jaw or pair of jaws;
a said clamp comprises a worm gear which engages the proximal ends of the jaws (5; 26), said proximal ends of the jaws (5; 26) configured as gear sectors containing at least one tooth each, said worm gear being rotatable and causing the actuation of said jaws (5; 26);
or
and wherein said worm gear is externally engageable by said actuator (10).

2. The surgical clamp system of claim 1, wherein both of the pair of jaws (5; 26) of said clamp (1; 25) are movable.

3. The surgical clamp system of claim 1, wherein one of the pair of jaws (5; 26) of said clamp (1; 25) is movable and the other of the pair of jaws (5; 26) of said clamp (1; 25) is stationary.

4. The surgical clamp system of anyone of claims 1 through 3, wherein the pair of jaws (5; 26) of said clamp (1; 25) are substantially parallel to each other in any position between the fully open and the fully closed positions.

5. The surgical clamp system of anyone of claims 1 through 3, wherein the pair of jaws (5; 26) of said clamp (1; 25) are only parallel to each other when fully closed, defining an angle when in any position other than fully closed.

## Patentansprüche

1. Chirurgisches Klemmsystem, mit:
einer Klemme (1; 25), die zwei Maulteile (5; 26) aufweist, die relativ zueinander aus einer vollständig offenen bis in eine vollständig geschlossene Stellung betätigbar sind;
einer Zuführ-/Entnahmevorrichtung (6), die über eine Bajonettverbindung (14; 23) lösbar mit der Klemme (1; 25) verbunden ist, wobei die Zuführ-/Entnahmevorrichtung (6) einen hohlen Schaft (7) mit einer längsverlaufenden hindurchgehenden Öffnung aufweist, wobei ein distaler Abschnitt der Öffnung so ausgestaltet ist, dass er mit der Klemme (1; 25) in Eingriff kommen kann, wobei die Zuführ-/Entnahmevorrichtung (6) durch einen Chirurgen bedienbar ist, um eine Funktion auszuführen, die aus der Gruppe ausgewählt ist, die ein Einbringen der Klemme (1; 25), ein Entnehmen der Klemme (1; 25) oder beides umfasst;
einem Betätigungsorgan (10), das mit der Zuführ-/Entnahmevorrichtung (6) zusammenwirkt, wobei das Betätigungsorgan (10) zumindest teilweise durch die längsverlaufende Öffnung der Zuführ-/Entnahmevorrichtung (6) zum Ineingriffkommen mit der Klemme (1; 25) einsetzbar ist, wobei das Betätigungsorgan durch den Chirurgen bedienbar ist, um die Maulteile (5; 26) der Klemme (1; 25) zu öffnen und zu schließen; und
wobei die Zuführ-/Entnahmevorrichtung (6) und das Betätigungsorgan (10) so bedienbar sind, dass die Maulteile (5; 26) der Klemme (1; 25) in einer beliebigen Stellung zwischen der vollständig offenen und der vollständig geschlossenen Stellung sicher verriegelt werden können, und zwar sowohl während und nachdem diese von der Zuführ-/Entnahmevorrichtung (6) gelöst wurde,
wobei die Klemme einen Schneckentrieb aufweist, der mit den proximalen Enden der Maulteile (5; 26) in Eingriff steht, wobei proximale Enden der Maulteile (5; 26) als Zahnradsektoren ausgebildet sind, die jeweils zumindest einen Zahn aufweisen, wobei der Schneckentrieb drehbar ist und die Betätigung der Maulteile (5; 26) bewirkt;
oder wobei die Klemme eine Schub-Zug-Stange aufweist, die an einem Ende mit zumindest einem der beiden Maulteile (5; 26) der Klemme verbunden ist, wobei eine Zahnstange mit einem Ende der Schub-Zug-Stange verbunden ist, das dem Ende entgegengesetzt ist, das mit zumindest einem der beiden Maulteile der Klemme verbunden ist, wobei ein Schneckentrieb mit der Zahnstange in Eingriff steht, wobei der Schneckentrieb bezüglich der Zahnstange drehbar ist, um eine axiale Verschiebung der Zahnstange und eine Betätigung des Maulteils oder beider Maulteile zu bewirken;
und wobei das Betätigungsorgan (10) außenseitig mit dem Schneckentrieb in Eingriff bringbar ist.

2. Chirurgisches Klemmsystem nach Anspruch 1, wobei beide Maulteile (5; 26) der Klemme (1; 25) beweglich sind.

3. Chirurgisches Klemmsystem nach Anspruch 1, wobei eines der beiden Maulteile (5; 26) der Klemme (1; 25) beweglich ist und das andere der beiden Maulteile (5; 26) der Klemme (1; 25) unbeweglich ist.

4. Chirurgisches Klemmsystem nach einem der Ansprüche 1 bis 3, wobei die beiden Maulteile (5; 26) der Klemme (1; 25) in jeder beliebigen Stellung zwischen der vollständig offenen und der vollständig geschlossenen Stellung im Wesentlichen parallel zueinander verlaufen.

5. Chirurgisches Klemmsystem nach einem der Ansprüche 1 bis 3, wobei die beiden Maulteile (5; 26) der Klemme (1; 25) nur dann parallel zueinander verlaufen, wenn sie vollständig geschlossen sind, und einen Winkel definieren, wenn sie in einer beliebigen anderen Stellung als der vollständig geschlossenen Stellung sind.

## Revendications

1. Système de clamp chirurgical comprenant :
un clamp (1 ; 25) ayant une paire de mâchoires (5 ; 26) pouvant être actionnées l'une par rapport à l'autre pour passer d'une position complètement ouverte à une position complètement fermée ;
un dispositif de placement/retrait (6), raccordé de manière amovible audit clamp (1 ; 25) par un raccord à baïonnette (14 ; 23), dans lequel ledit dispositif de placement/retrait (6) comporte un arbre creux (7) avec une ouverture longitudinale à travers celui-ci, une portion distale de l'ouverture étant configurée de manière à s'engager avec ledit clamp (1 ; 25), ledit dispositif de placement/retrait (6) pouvant être actionné par un chirurgien afin de remplir une fonction choisie dans le groupe comprenant le déploiement dudit clamp (1 ; 25), le retrait dudit clamp (1 ; 25), ou les deux ;
un actionneur (10) coopérant avec ledit dispositif de placement/retrait (6), ledit actionneur (10) pouvant au moins être inséré en partie au travers de l'ouverture longitudinale dudit dispositif de placement/retrait (6) afin d'engager ledit clamp (1 ; 25) et pouvant être actionné par le chirurgien afin d'ouvrir et de fermer les mâchoires (5 ; 26) dudit clamp (1 ; 25) ; et
dans lequel ledit dispositif de placement/retrait (6) et ledit actionneur (10) peuvent être actionnés afin de bloquer de manière sûre les mâchoires (5 ; 26) dudit clamp (1 ; 25) dans une position quelconque entre la position complètement ouverte et la position complètement fermée, à la fois pendant et une fois que le clamp a été détaché dudit dispositif de placement/retrait, dans lequel ledit clamp pouvant comprendre un engrenage à vis sans fin qui s'engage avec les extrémités proximales des mâchoires (5 ; 26), lesdites extrémités proximales des mâchoires (5 ; 26) étant configurées sous la forme de secteurs d'engrenage contenant au moins une dent chacun, ledit engrenage à vis sans fin pouvant tourner et provoquer l'actionnement desdites mâchoires (5 ; 26) ; ou
un poussoir raccordé, au niveau d'une extrémité, à au moins une de la paire de mâchoires (5 ; 26) dudit clamp;
un engrenage à crémaillère raccordé à une extrémité dudit poussoir à l'opposé de l'extrémité raccordée à au moins une de la paire de mâchoires dudit clamp;
un engrenage à vis sans fin s'engageant avec ledit engrenage à crémaillère, ledit engrenage à vis sans fin pouvant tourner par rapport audit engrenage à crémaillère afin de provoquer le déplacement axial dudit engrenage à crémaillère et l'actionnement de la mâchoire ou de la paire de mâchoires ;
et dans lequel ledit engrenage à vis sans fin peut être engagé de manière externe par ledit actionneur (10).

2. Système de clamp chirurgical selon la revendication 1, dans lequel les deux de la paire de mâchoires (5 ; 26) dudit clamp (1 ; 25) sont mobiles.

3. Système de clamp chirurgical selon la revendication 1, dans lequel l'une de la paire de mâchoires (5 ; 26) dudit clamp (1 ; 25) est mobile et l'autre de la paire de mâchoires (5 ; 26) dudit clamp (1 ; 25) est immobile.

4. Système de clamp chirurgical selon l'une quelconque des revendications 1 à 3, dans lequel la paire de mâchoires (5 ; 26) dudit clamp (1 ; 25) sont sensiblement parallèles l'une à l'autre dans une position quelconque entre la position complètement ouverte et la position complètement fermée.

5. Système de clamp chirurgical selon l'une quelconque des revendications 1 à 3, dans lequel la paire de mâchoires (5 ; 26) dudit clamp (1 ; 25) sont uniquement parallèles l'une à l'autre lorsqu'elles sont complètement fermées, en définissant un certain angle lorsqu'elles se trouvent dans une position quelconque différente de la position complètement fermée.
